# EUROPEAN PATENT SPECIFICATION

(11) **EP 1 589 088 B1**
(45) Date of publication and mention of the grant of the patent: **29.08.2007**
(21) Application number: 05104976.5
(22) Date of filing: 14.12.2000
(51) Int. Cl.: C09J 189/00, A61L 24/10

(54) **New bioadhesive composition comprising a bioadhesive polyphenolic protein, a polymer comprising carbohydrate groups, pharmaceutically acceptable fine filaments and uses thereof**
Neue bioadhäsive Zusammensetzung enthaltend ein bioadhäsives polyphenolisches Protein, ein Polymer mit Kohlenhydratgruppen, pharmazeutisch akzeptierbare feine Filamente und deren Verwendung
Nouvelle composition bioadhésive comprenant une protéine polyphénolique bioadhésive, un polymère avec des groupes carbohydrates, des filaments minces pharmaceutiquement acceptables et sa utilisation

(30) Priority: 17.12.1999 SE 9904650; 26.01.2000 US 178548 P; 10.03.2000 SE 0000799
(43) Date of publication of application: 26.10.2005
(62) Divisional of application: 00987904.0
(73) Proprietor: BioPolymer Products of Sweden AB, 441 24 Alingsas (SE)
(72) Inventor: Qvist, Magnus, 441 24, ALINGS S (SE); Hansson, Hans, Arne, 436 58, HOV S (SE)
(74) Representative: Bergstrand, Mikael Gudmundsson

(56) References cited:
- WO-A-88/03953
- WO-A-94/28937
- US-A- 5 015 677
- US-A- 5 030 230
- BENEDICT C V ET AL: "ADHESIVES FROM MARINE MUSSELS" ACS SYMPOSIUM SERIES, WASHINGTON, DC, US, 1989, pages 465-483, XP002938827 ISSN: 0097-6156
- "POLYPHENOLIC SUBSTANCE OF MYTILUS EDULIS: NOVEL ADHESIVE CONTAININFL-DOPA AND HYDROXYPROLINE" SCIENCE, AMERICAN ASSOCIATION FOR THE ADVANCEMENT OF SCIENCE, US, vol. 212, 29 May 1981 (1981-05-29), pages 1038-1040, XP002938828 ISSN: 0036-8075
- DEMING T J ET AL: "MUSSEL BYSSUS AND BIOMOLECULAR MATERIALS" CURRENT OPINION IN CHEMICAL BIOLOGY, CURRENT BIOLOGY LTD, LONDON, GB, vol. 3, 1999, pages 100-105, XP002938829 ISSN: 1367-5931
- YU M ET AL: "SYNTHETIC POLYPEPTIDE MIMICS OF MARINE ADHESIVES" MACROMOLECULES, AMERICAN CHEMICAL SOCIETY. EASTON, US, vol. 31, no. 15, 28 July 1998 (1998-07-28), pages 4739-4745, XP000769972 ISSN: 0024-9297
- WAITE J H: "MUSSEL GLUE FROM MYTILUS CALIFORNIANUS CONRAD: A COMPARATIVE STUDY" JOURNAL OF COMPARATIVE PHYSIOLOGY B. BIOCHEMICAL, SYSTEMIC, AND ENVIRONMENTAL PHYSIOLOGY, SPRINGER VERLAG, BERLIN, DE, vol. 156, 1986, pages 491-496, XP002938831 ISSN: 0174-1578
- STRAUSBERG R L ET AL: "PROTEIN-BASED MEDICAL ADHESIVES" TRENDS IN BIOTECHNOLOGY, ELSEVIER PUBLICATIONS, CAMBRIDGE, GB, vol. 8, February 1990 (1990-02), pages 53-57, XP002938832 ISSN: 0167-7799

## Description

The present invention relates to biodegradable compositions comprising adhesive, biocompatible polymers and methods used to cover surfaces and to attach structures to eye tissues, such as the cornea. Polyphenolic proteins isolated from mussels, referred to as "mussel adhesive protein" or "MAP", are used in conjunction with polysaccharides and pharmaceutically acceptable fine filaments, to achieve strong adhesive bonding. The invention also relates to a kit consisting of a MAP preparation, a preparation of polysaccharides which preferably are negatively charged, pharmaceutically acceptable fine filaments, and optionally an oxidising agent such as hydrogen peroxide nitroprusside ions or periodate ions, that is used for preparing a composition for covering and attaching structures to eye tissue, such as cornea.

### Background of the invention

The repair of traumatised structures on and in an eye and its adnexa is often troublesome. The use of sutures in e.g. the cornea is causing discomfort, deformations, distortions and may impair the visual acuity. Other eye components, such as the iris, lens structures and the retina are difficult or hardly possible to suture or to join with clips and related aids. Furthermore, sutures and clips do inevitably induce foreign body reactions and scar formation. The positioning of an implant in ocular structures, e.g. a partial-thickness or a penetrating keratoplasty or a prosthesis in the cornea, requires the use of elaborated techniques including the use of haptics to keep it retained in proper position, which may lead to irritations and adverse reactions. Accordingly, current methods for repairing structures on, at, and in an eye are associated with discomfort and the possibility of inducing permanent damage to the visual acuity. The use of a composition enabling structures with wet surfaces to be attached in desired position, remaining adherent for a predicted time period without causing any opacities in optically important components, or any deformation, scars or unacceptable foreign body reactions would therefore be highly desirable.

Polyphenolic proteins, preferentially isolated from mussels, are known to act as adhesives. Examples of such proteins can be found in e.g. US 4,585,585. Their wide use as adhesives has been hampered by problems related to the purification and characterisation of the adhesive proteins in sufficient amounts. Furthermore, the requirement for biocompatible suitable cross-linkers and other additives have limited their use. Chemicals, such as bifunctional conjugating compounds, and enzymes are commonly associated with toxic reactions and other biomedical side effects. Additionally, it is difficult to extensively purify enzymes with retained high activity, avoiding denaturation and adverse effects on cells, tissues or organs.

Mussel adhesive protein (MAP) is formed in a gland in the foot of byssus-forming mussels, such as the common blue mussel (Mytilus edulis). The molecular weight of MAP from Mytilis edulis is about 130.000 Dalton and it has been disclosed to consist of 75 - 80 closely related repeated peptide sequences. It has an unusual high proportion of hydroxy-containing amino acids such as hydroxyproline, serine, threonine, tyrosin, and the uncommon amino acid 3,4 dihydroxy-L-phenylalanine (Dopa) as well as lysine. It may be isolated either from natural sources or produced biotechnologically. US 5,015,677 as well as US 4,585,585 disclose that MAP has very strong adhesive properties after oxidation and polymerisation, e.g. by the activity of the enzyme tyrosinase, or after treatment with bifunctional reagents. It is very important in biomedical applications of an adhesive and coating composition to use bioacceptable and biodegradable components, which furthermore should not per se or due to contamination induce any inflammation or toxic reactions. Fillers, including collagens and polysaccharides, have been added to improve the mechanical properties in cases when MAP was used to bond tissues and structures together, further adding to the risk for immunological reactions.

It is also previously known that it is possible to use adhesive compositions based on MAP for ophthalmic purposes. Robin et al., Refractive and Corneal Surgery, vol. 5, p. 302 - 306, and Robin et al., Arch. Ophthalmol., vol. 106, p. 973 - 977, both disclose MAP-based adhesives comprising an enzyme polymiser. US 5,015,677 also describes a MAP-based adhesive containing a cross-linking agent and optionally a filler substance and a surfactant. Preferred cross-linking agents according to US 5,015,677 are enzymatic oxidising agents such as catechol oxidase and tyrosinase, but sometimes also chemical cross-linking agents such as glutaraldehyde and formaldehyde. Examples of fillers are proteins, such as collagen and albumin, and polymers comprising carbohydrate moieties, such as chitosan and hyaluronan. US 5,030,230 also relates to a bioadhesive comprising MAP, mushroom tyrosinase (cross-linker), SDS (sodium dodecyl sulfate, a surfactant) and collagen (filler). The bioadhesive is used to adhere a cornea prosthesis to the eye wall.

A major problem associated with known MAP-based bioadhesive compositions, despite the superior properties of MAP *per se,* is that some constituents, in particular the presently used cross-linking agents, can harm and/or irritate living tissue and cause toxic and immunological reactions. Chemical crosslinking agents, such as glutaraldehyde and formaldehyde, are generally toxic to humans and animals, and it is highly inappropriate to add such agents to a sensitive tissue, such as the eye. Enzymes, such as catechol oxidase and tyrosinase, are proteins, and proteins are generally recognised as potent allergens, especially in case they originate from a species other than the patient. Because of their oxidising and hydrolysing abilities, they can also harm sensitive tissue. Despite these serious drawbacks associated with the presently used cross-linkers, it has been regarded as necessary to include them in order to obtain sufficient curing of the bioadhesive.

WO-A1-94/28937 discloses protein-polymer conjugates as biocompatible adhesives.

Accordingly, there is a need for a MAP-based bioadhesive composition which overcomes these drawbacks and hence, does not harm or irritate sensitive tissues such as the eye.

### Summary of the invention

Now it has surprisingly been found that a non-irritating, non-allergenic and non-toxic composition can be obtained by providing a bioadhesive composition comprising a) a polyphenolic protein derived from byssus-forming mussels b) a polymer comprising carbohydrate groups, and c) pharmaceutically acceptable fine filaments. The bioadhesive composition does not contain any enzyme or chemical cross-linking agent. Optionally, the composition may contain an oxidising agent and/or a filler protein. Preferably, the composition is provided as a kit of at least two parts, wherein the polyphenolic protein and the polymer comprising carbohydrate groups are comprised in separate portions.

### Definitions

As disclosed herein, the terms "polyphenolic protein", "mussel adhesive protein" or "MAP" relates to a bioadhesive protein derived from byssus-forming mussels. Examples of such mussels are mussels of the genera Mytilus, Geukensia, Aulacomya, Phragmatopoma, Dreissenia and Brachiodontes. Suitable proteins have been disclosed in a plurality of publications, e.g. US-A-5,015,677, US-A-5,242,808, US-A-4,585,585, US-A-5,202,236, US-A-5149,657, US-A-5,410,023, WO 97/34016, and US-A-5,574,134, Vreeland et al., J. Physiol., 34: 1-8, and Yu et al., Macromolecules, 31: 4739-4745. They comprise about 30 - 300 amino acid residues and essentially consist of tandemly linked peptide units optionally separated by a junction sequence of 0 - 10 amino acids. A characteristic feature of such proteins is a comparatively high amount of positively charged lysine residues, and in particular the unusual amino acid DOPA (L-3,4-dihydroxyphenylalanine). A polyphenolic protein suitable for use in the present invention has an amino acid sequence in which at least 5 % and preferably 6-25 % of the amino acid resdues are DOPA. A few examples of typical peptide units are given below. However, it is important to note that the amino acid sequences of these proteins are variable and that the scope of the present invention is not limited to the exemplified subsequences below as the skilled person realises that bioadhesive polyphenolic proteins from different sources can be regarded as equivalent:
a) Val-Gly-Gly-DOPA-Gly-DOPA-Gly-Ala-Lys
b) Ala-Lys-Pro-Ser-Tyr-diHyp-Hyp-Thr-DOPA-Lys
c) Thr-Gly-DOPA-Gly-Pro-Gly-DOPA-Lys
d) Ala-Gly-DOPA-Gly-Gly-Leu-Lys
e) Gly-Pro-DOPA-Val-Pro-Asp-Gly-Pro-Tyr-Asp-Lys
f) Gly-Lys-Pro-Ser-Pro-DOPA-Asp-Pro-Gly-DOPA-Lys
g) Gly-DOPA-Lys
h) Thr-Gly-DOPA-Ser-Ala-Gly-DOPA-Lys
i) Gln-Thr-Gly-DOPA-Val-Pro-Gly-DOPA-Lys
j) Gln-Thr-Gly-DOPA-Asp-Pro-Gly-Tyr-Lys
k) Gln-Thr-Gly-DOPA-Leu-Pro-Gly-DOPA-Lys

As disclosed herein, the term "polymer comprising carbohydrate groups", relates to a naturally occurring or synthetic polymer comprising a plurality of carbohydrate groups. The polymers can be constituted of discrete carbohydrate groups joined by hydrocarbon chains, but preferably the polymers are polysaccharides, and still more preferably polysaccharides comprising charged groups. Examples of suitable polymers are heparin, chondroitin sulfate, chitosan and hyaluronan.

As disclosed herein, the term "pharmaceutically acceptable fine filaments" relates to thin fibres which can be used in sutures, preferably ophthalmic sutures. For the purposes of the present invention, the lengths of the fibres should not exceed 15 mm, and are typically within the range of 1 - 10 mm.

As disclosed herein, the term "filler protein" relates to a protein that is optionally added to the bioadhesive composition in order to obtain a bioadhesive composition that is adapted to special applications. Suitable filler proteins according to the present invention are collagen, casein, albumin, elastin, fibrin and fibronectin.

By the term "enzymatic oxidising agent" is meant an enzyme having the ability of oxidising MAP in order to promote full or partial cross-linking of MAP and/or polymers and/or filler proteins. Examples of such enzymes according to the state of the art include catechol oxidase and tyrosinase. A composition according to the present invention does not include an enzymatic oxidising agent. By the term "non-enzymatic oxidising agent" is meant a pharmaceutically acceptible oxidising agent which, at the doses employed, is non-toxic and non-irritating. Examples of such non-enzymatic oxidising agents are hydrogen peroxide and sodium nitroprusside.

By the term "chemical cross-linking agent" is meant a compound comprising at least two functional groups that are able to covalently couple to MAP and/or polymers and/or filler proteins. Examples of such compounds according to the state of the art include glutaraldehyde, formaldehyde, bis(sulfosuccinimidyl) suberate and 3,3'-dithiobis (sulfosuccinimidyl propionate). A composition according to the present invention does not include any chemical cross-linking agent.

A composition according to the present invention comprises three mandatory components, namely a) a bioadhesive polyphenolic protein, and b) a polymer comprising carbohydrate groups, and c) pharmaceutically acceptable fine filaments. It is preferred that the composition is supplied as a kit of parts, wherein the above mentioned mandatory components a) and b) are comprised in separate preparations. These preparations are mixed immediately before use. In the complete composition, the following concentrations have been found to be useful:

| Component | Concentration |
|---|---|
| | (mg/ml) |
| polyphenolic protein (component a) | 0.1 - 50, preferably 0.3 - 10 |
| polymer with carbohydrate groups | 0.1 - 50, preferably 0.3 - 30 |
| pharmaceutically acceptable fine | |
| filaments | 0.5 - 40, preferably 1 - 20 |

Optionally, a non-enzymatic oxidising agent can be included. One such agent is hydrogen peroxide, which typically can be included in an amount of 1 - 100 mg/ml, preferably about 10 mg/ml corresponding to 1 % (w/v). Other such agents are nitroprusside ions and periodate ions. Periodates, such as sodium periodate, are typically in a concentration of 2 mM counted on the final composition.

In case the composition is supplied as a kit of parts, each component is provided in the same or a higher concentration, but the above mentioned concentration ranges will be obtained upon mixing the component preparations with each other in order to prepare the final composition.

The present invention will now be further described in the following non-limiting example.

### Example

Purified MAP (0.81 mg/ml, BioPolymer Products AB) in 1 % lactic acid had its pH adjusted to 7.5 - 9.5. Heparin and laboratory chemicals were of highest available purity and purchased from Sigma and Merck. Ophthalmic sutures (10-0) were purchased from Ethicon, cut in small pieces ranging in length from 1-10 mm and eventually added to the MAP solution. The experiments were performed on anaesthetised rats according to the rules specified by the ethical permissions.

The cornea was deepithelialised. The cornea was carefully punched in the centre with aid of a trephine with a diameter of 3 mm. The tissue cylinder was slowly reacted to at least half of its thickness from the original cornea with aid of fine-pointed instruments under microscopic observation. Two small marks were made in the periphery of the central cornea plug. A solution, prepared immediately before use, of 20 µl MAP solution, 10 µl aqueous heparin solution, ophthalmic sutures (10mg/ml of the final composition)and 10 µl 6 % (w/v) aqueous hydrogen peroxide solution, was applied several times along the border between the plug and the remaining cornea. The cornea plug was then repositioned and gently kept in position. Adhesion was achieved between the central plug and peripheral part of the original cornea after 5-10 minutes, as very gently tested with aid of ophthalmic microsurgery instruments using an operating microscope.

Visual inspection and microscopy was performed during the subsequent 3 hours. There was good adhesion between the central corneal plug and the surrounding residuing cornea. The small defects in the interface zone were filled by the glue with its filaments.

The achieved results thus indicate that MAP in conjunction with heparin, an oxidising agent and very fine filaments could be used to safely glue a corneal plug back to its site of surgery and to fill and bridge defects in the tissue.

## Claims

1. A bioadhesive composition comprising
a) a bioadhesive polyphenolic protein derived from a byssus-forming mussel, which protein comprises 30 - 300 amino acids and consisting essentially of tandemly linked peptide repeats comprising 3-15 amino acid residues, wherein at least 5 % and preferably 6 - 25 % of the amino acid residues of said bioadhesive polyphenolic protein are DOPA;
b) a polymer comprising carbohydrate groups, such as heparin, chondroitin sulfate, chitosan and hyaluronan;
c) pharmaceutically acceptable fine filaments which are thin fibres suitable for being used in ophthalmic sutures, wherein the length of the fibres do not exceed 15 mm;
d) optionally a non-enzymatic oxidising agent such as hydrogen peroxide, nitroprusside ions or periodate ions; and optionally
e) a filler protein, such as collagen, casein, albumin, elastin, fibronectin and fibrin;
which composition does not comprise any enzymatic oxidising agent or chemical cross-linking agent.

2. A bioadhesive composition according to claim 1, for medical use.

3. Use of a composition according to claim 1 for preparing an ophtalmic adhesive which can be used to heal perforations, lacerations or incisions, to reattach the retina to the back of the eye, to repair or attach lenses, and to repair, construct, reconstruct and/or attatch corneal component parts.

## Patentansprüche

1. Bioadhäsive Zusammensetzung, umfassend:
a) ein bioadhäsives, polyphenolisches Protein, das von einer Muschel, die Byssus bildet, stammt, wobei dieses Protein 30 - 300 Aminosäuren umfasst und im Wesentlichen aus tandemartig verbundenen Wiederholungen von Peptiden, die 3 -15 Aminosäurereste umfassen, besteht, wobei mindestens 5 % und vorzugsweise 6 - 25 % der Aminosäurereste von besagtem, bioadhäsiven, polyphenolischen Protein DOPA sind;
b) ein Polymer, das Kohlenhydratgruppen wie zum Beispiel Heparin, Chondroitinsulfat, Chitosan und Hyaluronan umfasst;
c) pharmazeutisch annehmbare, dünne Filamente, die dünne Fasern, die dazu geeignet sind, in ophthalmischen Nähten verwendet zu werden, sind, wobei die Länge der Fasern 15 mm nicht überschreitet;
d) wahlweise ein nicht enzymatisches Oxidationsmittel wie zum Beispiel Wasserstoffperoxid, Nitroprussidionen oder Periodationen und wahlweise
e) ein Füllprotein wie zum Beispiel Kollagen, Casein, Albumin, Elastin, Fibronektin und Fibrin,
wobei diese Zusammensetzung kein enzymatisches Oxidationsmittel oder chemisches Vernetzungsmittel umfasst.

2. Bioadhäsive Zusammensetzung gemäß Anspruch 1 zur medizinischen Verwendung.

3. Verwendung einer Zusammensetzung gemäß Anspruch 1 zum Herstellen eines ophthalmischen Klebstoffs, der dazu verwendet werden kann, Perforationen, Lazerationen oder Inzisionen zu heilen, die Netzhaut wieder an der Rückseite des Auges zu befestigen, Linsen zu reparieren oder zu befestigen und Bestandteile der Hornhaut zu reparieren, aufzubauen, wieder aufzubauen und/oder zu befestigen.

## Revendications

1. Composition bioadhésive comprenant
a) une protéine polyphénolique bioadhésive dérivée d'une moule formant un byssus, laquelle protéine comprend 30 - 300 acides aminés et consistant essentiellement en des séquences de peptides liés répétées en tandem comprenant 3-15 résidus d'acides aminés, dans laquelle au moins 5 % et de préférence 6 à 25 % des résidus d'acides aminés de ladite protéine polyphénolique bioadhésive sont de la DOPA ;
b) un polymère comprenant des groupes carbohydrates, tels que l'héparine, la chondroitine, le sulfate, le chitosane et l'hyaluronane ;
c) des filaments fins acceptables pharmaceutiquement qui sont des fibres fines adaptées à être utilisées pour des sutures ophtalmiques, où la longueur des fibres ne dépasse pas 15 mm ;
d) en option, un agent oxydant non enzymatique tel qu'un péroxyde d'hydrogène, des ions nitroprussiate ou des ions périodate ; et en option
e) une protéine de remplissage, telle que du collagène, de la caséine, de l'albumine, de l'élastine, de la fibronectine et de la fibrine ;
laquelle composition ne comprend pas d'agent oxydant enzymatique ou d'agent de réticulation chimique quel qu'il soit.

2. Composition bioadhésive selon la revendication 1 destinée à un usage médical.

3. Utilisation d'une composition selon la revendication 1 pour préparer un adhésif ophtalmique qui peut être utilisé pour soigner des perforations, lacérations ou incisions, pour rattacher la rétine à l'arrière de l'oeil, pour réparer ou attacher des cristallins et pour réparer, construire, reconstruire et/ou attacher des parties de composantes cornéennes.
